# EUROPÄISCHE PATENTSCHRIFT

(11) **EP 1 189 614 B1**
(45) Veröffentlichungstag und Bekanntmachung des Hinweises auf die Patenterteilung: **04.06.2008**
(21) Anmeldenummer: 00942125.6
(22) Anmeldetag: 23.06.2000
(51) Int. Cl.: A61K 31/4525, A61K 9/20, A61P 25/24, A61K 9/08

(54) **FESTE PAROXETIN ENTHALTENDE ZUBEREITUNGEN**
SOLID PREPARATIONS CONTAINING PAROXETINE
PREPARATIONS SOLIDES CONTENANT DE LA PAROXETINE

(30) Priorität: 02.07.1999 DE 19930454
(43) Veröffentlichungstag der Anmeldung: 27.03.2002
(73) Patentinhaber: Abbott GmbH & Co. KG, 65205 Wiesbaden (DE)
(72) Erfinder: ROSENBERG, Jörg, D-67158 Ellerstadt (DE); BREITENBACH, Jörg, D-68199 Mannheim (DE); LIEPOLD, Bernd, D-68161 Mannheim (DE)
(74) Vertreter: Kinzebach, Werner
(86) Internationale Anmeldenummer: PCT/EP2000/005848
(87) Internationale Veröffentlichungsnummer: WO 2001/001956

(56) Entgegenhaltungen:
- EP-A- 0 214 092
- EP-A- 1 027 886
- WO-A-99/00131
- WO-A-99/26625
- DE-A- 4 406 462
- D.Q.M. CRAIG: "A review of thermal methods used for the analysis of the crystal form, solution thermodynamics and glass transition behaviour of polyethylene glycols"" THERMOCHIMICA ACTA, Bd. 248, 1995, Seiten 189-203,

## Beschreibung

Die vorliegende Erfindung betrifft ein Verfahren zur Herstellung fester oder halbfester Zubereitungen von Paroxetin oder einem seiner physiologisch aktiven Salze in Form einer molekulardispersen Verteilung in einem pharmazeutisch akzeptablen Matrixmaterial.

Paroxetin ist der generische Name für (-)-trans-4-(4'-fluorphenyl)-3-(3,4'-methylendioxiphenoximethyl)-piperidin, welches beispielsweise in der US-A 4 007 196 beschrieben ist.
Paroxetin gehört zur Klasse der 5-Hydroxytryptamin-Inhibitoren und wird als Antidepressivum verwendet.

Aufgrund seiner Basizität wird Paroxetin zur therapeutischen Anwendung in Form seiner Säureadditionssalze eingesetzt, insbesondere in Form des physiologisch besonders akzeptablen Hydrochlorids. Allerdings weist Paroxetin-Hydrochlorid-Anhydrat eine Neigung zur Polymorphie auf. So werden in der DE-C 196 03 797 vier polymorphe Formen des Paroxetin-Hydrochlorid-Anhydrats beschrieben. Polymorphe Formen sind in der therapeutischen Anwendung aber insofern problematisch, als unterschiedliche Polymorphe unterschiedliche Löslichkeiten und Unterschiede in der Bioverfügbarkeit zur Folge haben können.

Eine Lösung des Polymorphie-Problems kann darin bestehen, den Wirkstoff in amorpher Form bereitzustellen. So ist in der WO 99/16440 die Herstellung amorpher, also nicht-kristalliner, Paroxetin-Hydrochlorid-Formulierungen durch Lösen in einer Hydroxyl-Gruppen enthaltenden Verbindung wie Ethanol und anschließende Entfernung dieser Verbindung beschrieben. Ebenso wird in der EP-A 0 810 224 die Herstellung von amorphem Paroxetin-Hydrochlorid durch Lösen des Wirkstoffs in Wasser oder einem niederen Alkohol mit anschließender Entfernung des Lösungsmittels, beispielsweise durch Sprühtrocknung, beschrieben.

Feststoffdispersionen, also homogene feinstdisperse Phasen, von zwei oder mehreren Feststoffen sowie ihr Sonderfall der sogenannten "festen Lösungen" (molekulardisperse Systeme) sowie ihr Einsatz in der pharmazeutischen Technologie sind allgemein bekannt (vgl. Chiou und Riegelman, J. Pharm. Sci., 60, 1281-1300 (1971)).

In der WO 99/00131 ist die Herstellung fester Dispersionen schwer wasserlöslicher Substanzen mit Hilfe eines Lösemittelverfahrens oder eines Schmelzeverfahrens beschrieben. Danach kann beispielsweise eine feste Dispersion von Paroxetin-Hydrochlorid in einem festen Trägermaterial erzeugt werden, indem die freie Paroxetin-Base in Gegenwart des Trägermaterials geschmolzen und dann die Schmelze mit trockenem Chlorwasserstoffgas durchspült wird. Anschließend wird die Schmelze beispielsweise durch Stehenlassen über Nacht auf Raumtemperatur abgekühlt und gemahlen. Allerdings ist die in diesem Dokument beschriebene Verfahrensweise eher auf den Labormaßstab beschränkt und läßt im Hinblick auf die Homogenität der Mischungen noch zu wünschen übrig. Hinzu kommt, daß das chemisch sehr reaktive Chlorwasserstoffgas mit den Hilfsstoffen reagieren und toxikologisch bedenkliche Reaktionsprodukte bilden kann.

Aus der EP-A 665 009 ist bekannt, daß man den Kristallzustand von Wirkstoffen durch Verarbeitung in einem Schneckenextruder ändern kann, wobei die Wirkstoffe im wesentlichen ohne weitere Hilfsstoffe verarbeitet werden.

Weiterhin ist aus der EP-A 760 654 bekannt, daß man Säureadditionssalze direkt mit Hilfe eines Schmelzextrusionsverfahrens durch Umsetzung der freien Base in Gegenwart eines Salzes herstellen kann.

Aus der WO 99/26625 sind Formulierungen von Paroxetin bekannt, bei denen der Wirkstoff in einem Copolymer gelöst und mit einem geschmolzenen Polymer vermischt wird. Solche Formulierungen können auch extrudiert werden. Allerdings neigen solche Formulierungen aufgrund der Verwendung eines Cosolvens zur Rekristallisation.

Verbesserte Zubereitung von Paroxetin und dessen physiologisch akzeptablen Salzen helfen zum einen das Problem der Polymorphie vermeiden zum anderen weisen sie aber auch eine verbesserte Löslichkeit und Lagerstabilität des an sich schlecht löslichen Wirkstoffs Paroxetin auf. Die Aufgabe der vorliegenden Erfindung war es, ein vereinfachtes Verfahren zur Herstellung solcher Zubereitungen zur Verfügung zu stellen.

Demgemäß wurden feste Zubereitungen des Paroxetins und seiner physiologisch akzeptablen Salze gefunden, in denen der Wirkstoff molekulardispers in einem pharmazeutisch akzeptablen Trägermaterial eingebettet ist, welches ein vollsynthetisches Polymer mit einer Glasübergangstemperatur im wasserfreien Zustand von >90°C enthält.

Die Zubereitungen können auch halbfest sein, jedoch sind feste Formen bevorzugt.

Als pharmazeutisch akzeptable Salze des Paroxetins kommen neben Salzen wie beispielsweise dem Fumarat oder dem Maleat vor allem das Hydrochlorid sowie das entsprechende Hydrochlorid-Anhydrat in Betracht.

Als pharmazeutisch akzeptable Matrix- oder Trägermaterialien kommen grundsätzlich alle Materialien in Betracht, die sich mit Hilfe eines Schmelzeverfahrens zu einer homogenen Matrix mit dem Wirkstoff verarbeiten lassen.

Geeignete Matrixpolymere weisen im wasserfreien Zustand eine Glasübergangstemperatur >90°C, bevorzugt >90 bis 110°C, auf und sind vollsynthetische Polymere. In Betracht kommen vor allem thermoplastisch verarbeitbare wasserlösliche Polymere wie die Homo- oder Copolymere des N-Vinylpyrrolidons mit K-Werten nach Fikentscher im Bereich von 19 bis 100.

Bevorzugte Matrixmaterialien sind Polyvinylpyrrolidone oder Copolymere aus N-Vinylpyrrolidon und Vinylacetat wie VP/VAc 60/40 (Copovidon).

Weiterhin können der Matrix noch übliche pharmazeutische Hilfsstoffe wie Füllstoffe, Trennmittel, Sprengmitttel, Stabilisatoren, Mittel zur Geschmacksverbesserung, Antioxidantien oder Farbstoffe zugegeben werden.

Die Zubereitungen können das Paroxetin oder eines seiner Salze in Mengen von 0,1 bis 50 Gew.-%, bevorzugt 5 bis 30 Gew.-%, bezogen auf das Gesamtgewicht der Zubereitung, enthalten.

Die Herstellung der Zubereitungen erfolgt mit Hilfe eines Schmelzeverfahrens, durch Herstellung und Verarbeitung der Schmelze mit Hilfe eines Schneckenextruders.

Die Herstellung kann derart erfolgen, daß zunächst eine pulverförmige Vormischung sämtlicher Einsatzstoffe hergestellt und in einen Schneckenextruder eingebracht wird. Unter Einbringung von Scherkräften und thermischer Energie wird diese Vormischung zu einer homogenen Schmelze verarbeitet und anschließend der Formgebung unterworfen. Die Herstellung der Schmelze erfolgt vorzugsweise bei Temperaturen im Bereich von 80 bis 100°C, bevorzugt 80 bis 150°C. Man kann auch zunächst nur die Matrixmaterialien aufschmelzen und dann den Wirkstoff durch geeignete Vorrichtungen zudosieren.

Als Schneckenextruder wird vorzugsweise ein gleichsinnig drehender Doppelschneckenextruder eingesetzt. Die so erzeugte homogene Schmelze kann entweder durch eine Düse oder eine Lochplatte extrudiert werden, oder auch über den offenen Extruderkopf gefördert werden und dabei gegebenenfalls durch im Schneckenkanal angeordneten Mahlelemente direkt als Granulat gefördert werden. Weiterhin kann die Formgebung nach den üblichen Abschlagtechniken erfolgen, beispielsweise durch Heißabschlag oder Kaltabschlag oder durch Druckluftabschlag.

Die Formgebung der extrudierten, noch thermoplastischen Schmelze kann auch dergestalt erfolgen, daß der extrudierte Strang zwischen gegenläufig rotierende Kalanderwalzen mit Vertiefungen geführt wird, wobei direkt Tablettenformen erzeugt werden können.

Die Herstellung der Zubereitungen erfolgt vorzugsweise in Abwesenheit von Lösungsmitteln. Sollten die Einsatzstoffe jedoch Lösungsmittel enthalten, so werden diese im Extruder durch Anlegen eines Vakuums entfernt. Auf diese Weise läßt sich auch im eingesetzten Wirkstoff noch enthaltenes Kristallwasser entfernen. Als Lösungsmittel eignen sich beispielsweise flüchtige organische Lösungsmittel oder Wasser.

Gemäß einer besonders bevorzugten Ausführungsform der Erfindung erfolgt die Herstellung des Paroxetin-Salzes durch gemeinsame Verarbeitung der freien Base Paroxetin zusammen mit einer zur Bildung eines entsprechenden Säureadditionsssalzes geeigneten Verbindung sowie den entsprechenden Matrixmaterialien mit Hilfe eines Schmelzextrusionsprozesses in einem Schneckenextruder. Bevorzugt wird zur Herstellung des entsprechenden Hydrochlorids als salzbildende Komponente Ammoniumchlorid eingesetzt.

Bevorzugt sind erfindungsgemäß Zubereitungen mit schneller Freisetzung des Wirkstoffs. Schnell freisetzend bedeutet, daß die Wirkstofffreisetzung, gemessen in einer Paddle-Apparatur, bei pH 1,2, 50 Upm und 37°C, nach 30 min. mindestens 80 % beträgt.

Die festen Zubereitungen enthalten den Wirkstoff in molekulardisperser Form in einer Matrix eingebettet. Die Matrix verhält sich wie ein echtes Lösungsmittel, d.h. jedes Wirkstoffmolekül wird von Molekülen der Matrixmaterialien umgeben. Optisch ist dies daran zu erkennen, daß die entstehenden erkalteten Schmelzen transparent sind. Dieser molekulardisperse Zustand der erkalteten Schmelze ist zudem auch thermodynamisch stabil, d.h. es kommt nicht zu Rekristallisationsvorgängen. Als Folge der molekulardispersen Verteilung des Wirkstoffs in der Matrix weisen die Zubereitungen eine schnelle gleichförmige Wirkstofffreisetzung auf: nach 30 min. ist der Wirkstoff im wesentlichen aus der erstarrten Schmelze freigesetzt.

Untersucht man die extrudierten Schmelzen mit Hilfe der Differential Scanning Calorimetry (DSC), so sind keine Schmelzsignale im Bereich des Wirkstoff-Schmelzpunkts mehr zu beobachten. Im Falle von polymeren Matrixmaterialien lassen sich lediglich breite Polymer-Glasübergangsstufen erkennen.

Erfindungsgemäß kann auch bereits amorphes Paroxetin oder dessen Salze eingesetzt werden. Die amorphen Formen lösen sich schneller in der Matrix, da keine Gitterenergie zum Schmelzen aufgebracht werden muß. Damit kann die Verarbeitung bei niedrigeren Temperaturen erfolgen.

Die Zubereitungen sind zudem auch stabil gegen Feuchtigkeitsaufnahme, d.h. es findet keine Rekristallisation statt. Dies ist umso erstaunlicher, als extreme hydrophile Polymere eingesetzt werden. Die Produkte weisen auch eine verbesserte Lagerstabilität auf. Überraschenderweise läßt sich das Paroxetin auch trotz der empfindlichen Acetal-Schutzgruppe unzersetzt extrudieren. Dies ist umso überraschender als PVP und seine Copolymere einen sauren pH aufweisen.

Die Zubereitungen können in Form von Granulaten erhalten werden und als solche in Kapseln abgefüllt oder zu Tabletten verpreßt werden, oder wie bereits geschildert, direkt zu Tablettenformen kalandriert werden oder auch als halbfeste Zubereitungen in Kapseln abgefüllt werden.

### Beispiele

Verarbeitet wurden Pulvervormischungen der folgenden Zusammensetzung, wobei jeweils wasserfreies Paroxetin-Hydrochlorid eingesetzt wurde:

### Beispiel 1

| | |
|---|---|
| Paroxetin-Hydrochlorid | 30 Gew.-% |
| Copovidon feindisperse Kieselsäure (1 Gew.-% bez. auf Wirkstoff/Polymer) | 70 Gew.-% |

Die Pulvervormischung wurde in einem Doppelschneckenextruder mit einem Förderschneckendurchmesser von 16 mm bei einer Materialtemperatur von 145°C aufgeschmolzen und extrudiert. Die erhaltene leicht gelbliche transparente Schmelze blieb auch nach Abkühlen transparent. Auch nach 9 Monaten Lagerung bei 40°C und 45 % relativer Luftfeuchte blieb die Transparenz erhalten.

### Beispiel 2

Eine Mischung gemäß Beispiel 1 wurde analog durch eine Rundstrangdüse mit einem Durchmesser von 3 mm ausgetragen. Zur Bestimmung der Wirkstofffreisetzung wurden die erkalteten, transparenten Strangstücke in Stücke mit einem Gewicht von 133 mg abgeteilt (Paroxetin-Hydrochlorid-Gehalt von 40 mg). Die Freisetzung wurde gemäß USP XXII bei pH 1,2, 50 Upm und 37°C in einer Paddle-Apparatur bestimmt:

| Zeit [min] | Wirkstofffreisetzung [Gew.-%] |
|---|---|
| 0 | 0 |
| 5 | 19 |
| 10 | 42 |
| 20 | 82 |
| 30 | 96 |
| 60 | 99 |

### Beispiel 3

### Herstellung von Tabletten

Hergestellt wurden bikonvexe Tabletten mit einem Durchmesser von 9 mm und einem Tablettengewicht von 200 mg durch Verpressen der Einsatzstoffe in einer konventionellen Tablettenpresse (Exzenterpresse Fette E2) bei einem Preßdruck von 6,5 kN. Die Tablette wies die nachstehende Zusammensetzung auf:

| | |
|---|---|
| Paroxetinhydrochlorid-Extrudat gem. Bsp. 1 | 38 Gew.-% |
| Mikrokristalline Cellulose | 15 Gew.-% |
| Calciumhydrogenphosphat (wasserfrei) | 35 Gew.-% |
| Na-Croscarmellose | 10 Gew.-% |
| Hochdisperses Siliciumdioxid | 1 Gew.-% |
| Mangnesiumstearat | 1 Gew.-% |

Die Tabletten waren in Wasser bei 37°C in 5 min komplett verfallen.

## Patentansprüche

1. Verfahren zur Herstellung einer festen oder halbfesten Zubereitung von Paroxetin oder einem seiner physiologisch akzeptablen Salze in Form einer molekulardispersen Verteilung des Paroxetins in einem pharmazeutisch akzeptablen Matrixmaterial, welches ein vollsynthetisches Polymer mit einer Glasübergangstemperatur im wasserfreien Zustand > 90°C enthält, **dadurch gekennzeichnet, dass** man das Paroxetin oder eines seiner Salze und das Matrixmaterial in einem Schneckenextruder zu einer homogenen Schmelze vermischt und anschließend der Formgebung unterwirft.

2. Verfahren nach Anspruch 1 zur Herstellung einer Zubereitung des Paroxetin-Hydrochlorids, **dadurch gekennzeichnet, dass** man Paroxetin mit Ammoniumchlorid und den Matrixmaterialien zu einer homogenen Schmelze verarbeitet.

3. Verfahren nach Anspruch 2, **dadurch gekennzeichnet, dass** man amorphes Paroxetin einsetzt.

## Claims

1. A process for producing a solid or semisolid preparation of paroxetine or one of its physiologically acceptable salts in the form of a molecular dispersion of paroxetine in a pharmaceutically acceptable matrix material which comprises a completely synthetic polymer having a glass transition temperature of >90°C in the anhydrous state, which comprises the paroxetine or one of its salts and the matrix material being mixed to give a homogeneous melt in an extruder and subsequently being shaped.

2. The process according to claim 1 for producing a paroxetine hydrochloride preparation, wherein paroxetine is processed with ammonium chloride and the matrix materials to give a homogeneous melt.

3. The process according to claim 2, wherein amorphous paroxetine is employed.

## Revendications

1. Procédé pour produire une préparation solide ou semi-solide de paroxetine ou de l'un de ses sels physiologiquement acceptables sous forme d'une répartition dispersée à l'échelle moléculaire de paroxetine dans un matériau de matrice pharmaceutiquement acceptable, qui contient un polymère totalement synthétique ayant une température de transition vitreuse à l'état anhydre > 90°C, **caractérisé en ce que** l'on mélange la paroxetine ou l'un de ses sels et le matériau de matrice dans une extrudeuse à vis en une masse fondue homogène puis on les soumet à la mise en forme.

2. Procédé selon la revendication 1 pour produire une préparation de chlorhydrate de paroxetine, **caractérisé en ce que** l'on transforme la paroxetine avec du chlorure d'ammonium et le matériau de matrice en une masse fondue homogène.

3. Procédé selon la revendication 2, **caractérisé en ce que** l'on utilise de la paroxetine amorphe.
